# EUROPEAN PATENT SPECIFICATION

(11) **EP 2 163 219 B1**
(45) Date of publication and mention of the grant of the patent: **11.04.2012**
(21) Application number: 08016174.8
(22) Date of filing: 15.09.2008
(51) Int. Cl.: A61B 19/02, A61L 2/26

(54) **Cassette for storage of medical instruments**
Kassette zur Aufbewahrung medizinischer Instrumente
Cassette pour stocker des instruments médicaux

(43) Date of publication of application: 17.03.2010
(73) Proprietor: Straumann Holding AG, 4002 Basel (CH)
(72) Inventor: Junk, Thomas, D-81667 München (DE); Thomaser, Astrid, D-81371 München (DE); Aidehag, Frederik, D-80469 München (DE); Bigio, Robin, D-80469 München (DE)
(74) Representative: Schaad, Balass, Menzl & Partner AG

(56) References cited:
- US-A- 2 955 705
- US-A- 3 133 635
- US-A- 4 135 868
- US-A- 4 881 705
- US-A- 4 959 199
- US-A- 5 024 326
- US-A- 5 031 768
- US-A- 5 384 103
- US-A- 5 525 314
- US-A- 5 681 539
- US-A- 5 848 693

## Description

The invention relates to a cassette for storage of a medical instrument.

Cassettes for storage of medical instruments represent well-known systems for storage, organization, presentation and sterilization of said instruments. Most of these cassettes comprise at least one tray in which the medical instruments are accommodated and a cover to close said tray.

Such a cassette is disclosed for instance in US 5,525,314. A therein described surgical tool container comprises an outer case having separable lid and base portions and with a tool-holding tray removably inserted within the base portion. Elastomeric grommets and resilient finger brackets are secured on the tool-holding tray. The elastomeric grommets and resilient finger brackets are provided for accommodating shanks of the surgical tools and are removably held by these elements due to frictional engagement therewith.

A further cassette is disclosed in US 5,384,103. The instrument tray described in the document includes a tray and an optional cover for storage, transport and sterilization of medical instruments. Interchangeably disposed along the surface of the tray and cover is a plurality of support members, each including variously dimensioned notched receptacles for receiving medical instruments. The support members are made of resilient material which is capable of replaceably accommodating medical instruments.

US 5,031,768 discloses a hinged instrument tray and disposable receptacle for contaminated waste, comprising a tray means for organizing and holding a plurality of items and a locking means. The tray means is formed in two halves, which are joined by a hinge, and comprises several compartments, each having means for holding and organizing at least one type of item. The preamble of claim 1 is based on this document.

US 2,955,705 refers to a transparent container for rigid, elongated articles, such as hypodermic syringes. The container comprises a flat, rectangular shaped base and a dished, transparent cover associated with said base. As a means for resiliently and releasably gripping a syringe, a bottom wall of the base is provided with a pair of gripping supports, which are molded integrally with the bottom wall and upstanding therefrom.

US 5,024,326 relates to a medical instrument holder and sharps disposal device, wherein a pair of plastic container body halves are provided to be manipulated between opened and closed positions by associated hinge means and a mechanical lock. A medical instrument rest is provided on one of the body halves for positioning medical instruments thereon when the container is in its open position.

Further examples of such cassettes are described e.g. in DE-A-10 2005 047 099, US 5,979,643, US 2007/0119737, WO 2005/053597, US 5,346,667, WO 2006/071180 and WO 00/57810.

In the cassettes of the prior art the resilient tool-holders are secured to the base of the tray by snugly fitting in corresponding openings of the tray. In this way fluids, bacteria, dirt etc. may enter into gaps between the base and the resilient material. Thus the sterility of the medical instruments might be at risk.

Therefore, it is an object of the present invention to provide a cassette for medical instruments which allows an optimized storage of these instruments under clean and sterile conditions.

This problem is solved by the cassette according to claim 1. Preferred embodiments are provided with the features contained in the dependent claims.

The inventive cassette for medical instruments comprises at least one tray and a cover, where the tray is provided with a holding means fixed to a rigid basic structure of the tray. The holding means comprises at least one cutout for retaining of medical instruments. In the inventive cassette the holding means is fixed to the rigid basic structure of the tray in a gap-free manner by means of molding, welding or gluing. In this way, bacteria, fluids, dirt etc. cannot enter in a connecting portion between the holding means and the rigid basic structure of the tray. Thus the medical instruments can be stored under clean and sterile conditions for longer times than in cassettes according to the prior art.

The rigid basic structure of the tray is provided with a socket having a base socket portion which is raised with respect to a tray base of the tray and the holding means is fixed on a top side of said base socket portion. Consequently, the connecting portion between the holding means and the rigid basic structure of the tray is arranged above a base level of the tray so that even if small amounts of undesired fluids may reach into the tray the connecting portion will be above an upper fluid level and not in contact with the fluid.

In a preferred embodiment all edges and corners between said base socket portion and the tray base are rounded in order to avoid adherence of impurities in these regions and to simplify cleaning procedures.

Particularly preferred embodiments of the present invention are described below and illustrated in the drawings in which purely schematically:
- Fig. 1: shows in a perspective view a first embodiment of the inventive cassette for accommodating medical instruments;
- Fig. 2: shows in a perspective exploded view the first embodiment of the inventive cassette in which a first and below a second tray is visible;
- Fig. 3: shows in a perspective view the first embodiment of the inventive cassette in which from left to right the cover (upside down), the second tray and the first tray can be seen;
- Fig. 4: shows in a perspective view the first embodiment of the inventive cassette without the cover where the first tray is set on the second tray;
- Fig. 5: shows a second embodiment of the inventive cassette in which the tray is slid in the cover;
- Fig. 6: shows in a perspective view the second embodiment of the inventive cassette in an upside down position;
- Fig. 7: shows in a perspective view the second embodiment of the inventive cassette where the tray is partially pulled out of the cover;
- Fig. 8: shows the second embodiment of the inventive cassette where the tray and the cover are presented side by side;
- Fig. 9: shows in a perspective view a third embodiment of the inventive cassette with a cover above a first and a second tray;
- Fig. 10: shows in a side view the second embodiment of the inventive cassette;
- Fig. 11: shows in a further side view the third embodiment of the inventive cassette;
- Fig. 12: shows in a perspective exploded view (from top to bottom) the cover, the first tray and the second tray;
- Fig. 13: shows in a perspective view the cover of the third embodiment of the inventive cassette in an upside down position;
- Fig. 14: shows in a perspective view the first tray of the third embodiment of the inventive cassette; and
- Fig. 15: shows in a perspective view the second tray of the third embodiment of the inventive cassette.

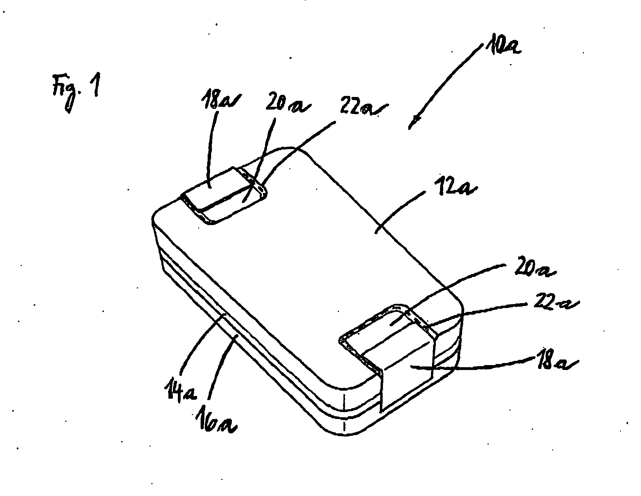

The first embodiment of the inventive cassette 10a - as shown in Fig. 1 - comprises a cover 12a arranged on top of a first tray 14a which in turn is arranged above a second tray 16a. The first tray 14a is enclosed in the cassette 10a in a sandwich-like arrangement. The cassette 10a is substantially of cubical configuration. It should be mentioned that the extension 'a' in the reference numerals refers to elements of the first embodiment. Although of similar function and / or construction the elements of the two later described embodiments are provided with the corresponding extensions 'b' and 'c', respectively.

The second tray 16a comprises at each longitudinal side spring clasps 18a which engage in the closed state of the cassette 10a with matching impressions 20a of the cover 12a. Due to the elasticity and the angular shape of the clasps 18a easy closing and removal of the cover 12a from the first and the second tray 14a, 16a are allowed. For closing of the cassette 10a the clasps 18a are deviated in direction to the cover 12a and snapped into the impressions 20a. By pulling the clasps 18a away from the impressions 20a of the cover 12a, the cover 12a as well as the first tray 14a can be completely lifted from the second tray 16a.

In preferably inclined transition portion extending from the impression 20a of the cover 12a to the top surface of the cover 12a elongated outer openings of a cover perforation 22a are formed. The cover perforation 22a allows flow of a sterilization medium through the cover perforation 22a into the inner of the cassette 10a.

The cover 12a, the first tray 14a and the secondary tray 16a of the cassette 10a comprise a rigid basic structure 23a preferably made of rigid plastics or metal.

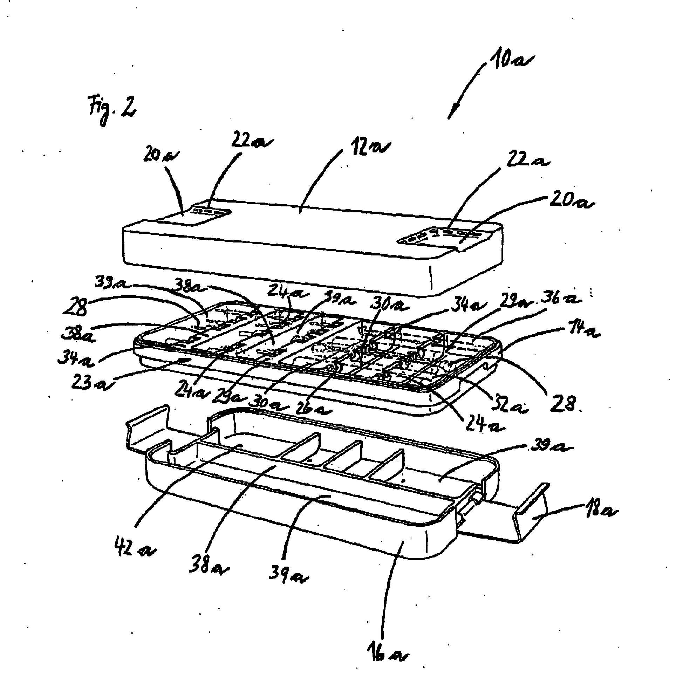

As shown in Figs. 2 to 4 the first tray 14a is provided with a number of holding means 24a which have cutouts 26a for retaining medical instruments 28 like drills, screwdriver and / or wrenches. The holding means 24a are attached to the rigid basic structure 23a of the first tray 14a in a gap-free manner by molding, welding or gluing. This feature can be directly identified by a user of the cassette 10a. Due to the gap-free connection between the holding means 24a and the rigid basic structure 23a bacteria, fluids etc. cannot enter between these two elements.

In Figs. 2 to 4 on the right hand side of the first tray 14a two comb-like holding means 24a for longer medical instruments 28 are arranged. The two holding means 24a are of identical construction and comprise cutouts 26a of substantially rectangular and/or rounded cross-section. Both holding means 24a are arranged parallel to each other and are spaced by a certain distance so that hafts of the medical instruments 28 can be holded.

The size of the cutouts 26a is slightly smaller than the diameter of the shaft of the medical instruments 28 in order to retain the later securely in the holding means 24a. The holding means 24a are preferably made of resilient plastic material of medical grade which can resist sterilization procedures for the medical instruments 28.

The medical instruments 28 can be snap-fitted into the cutouts 26a under deformation of the resilient holding means 24a. In its final position the medical instruments 28a are removably held in the cutout 26a due to the shape of the resilient holding means 24a in their unloaded state and/or friction between the medical instruments 28 and the holding means 24a.

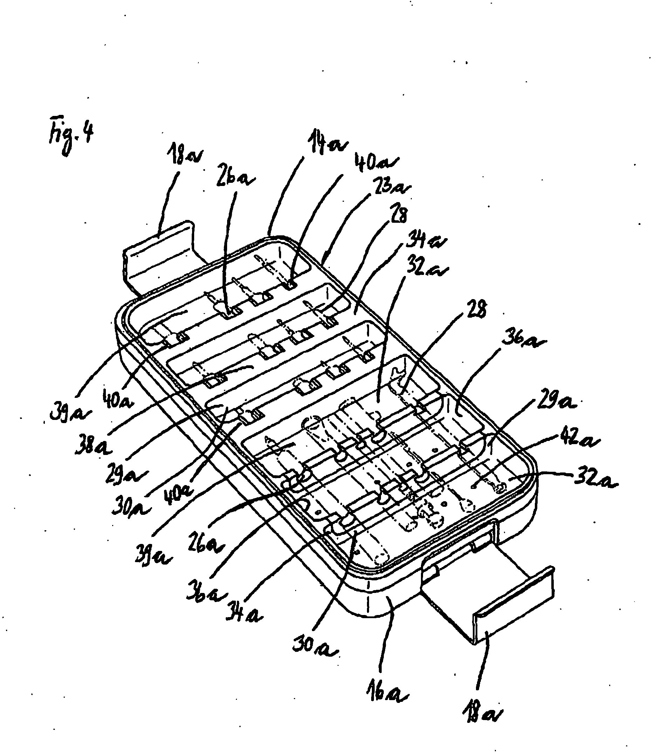

It is shown in Fig. 4 that the comb-like holding means 24a are base-sidedly of rounded shape and are fixed on sockets 29a of the rigid basic structure 23a. The sockets 29a comprise a base socket portion 30a raised with respect to a tray base 32a of the first tray 14a and lateral socket portions 34a protruding from lateral side walls 36a of the first tray 14a. Because of the height of the base socket portion 30a and the lateral socket portion 34a a connecting portion between the holding means 24a and the rigid basic structure 23a is raised with respect to the surfaces of the tray base 32a and of the lateral side walls 36a such that contacts of the connecting portion with dirt, bacteria and undesired fluids etc. is avoided. In this way the medical instruments 28 are stored under better conditions regarding cleanness and sterility.

The sockets 29a of the rigid basic structure 23a can integrate further substantially rigid protrusions extending from said base socket portions 30a and/or lateral socket portions 34a.

Such further protrusions are apt to provide the holding means 24a with an increased stiffness, particularly in those cases when a greater height and/or thickness of the resilient holding means 24a is required for the purpose of achieving an optimal engagement and accommodation of the medical instruments 28.

The above embodiments of the sockets 29a integrating further substantially rigid protrusion may prove especially advantageous for holding of medical instruments 28 which have greater diameters and cross sections, respectively.

For smaller medical instruments 28, like drills etc. single-instrument holding means 24a of smaller configuration compared to the comb-like holding means 24a are provided integrally in three, laterally arranged inner walls 38a of the first tray 14a. These holding means 24a are fixed in a gap-free manner to inner wall cutouts 40a. They are open to a top side of the inner walls 38a and to a lateral side of the inner walls 38a. The holding means 24a arranged in the inner wall cutouts 40a form inner resilient liners of the inner wall cutouts 40a. Due to their top side opening medical instruments 28 can be snapped in the single-instrument holding means 24a from above. Since all single-instrument holding means 24 are also open to the same lateral side of the inner walls 38a therein held medical instruments 28 point in the same direction.

The single-instrument holding means 24 are also formed above and laterally of sockets 29a. In this way medical instruments 28 as well as connecting portions between the holding means 24a and the sockets 29a, in particular the base and lateral socket portions 30a, 34a, does not come in contact with surfaces of the tray base 32a and the lateral side walls 36a.

The comb-like holding means 24a and the inner walls 38a separate six compartments 39a within the first tray 14a. All edges and corners of these compartments 39a are at least base-sidedly rounded in order to avoid undesired adhesion of impurities and complicated cleaning procedures. This is particularly true for all edges and corners between the base socket portions 30a and the tray base 32a.

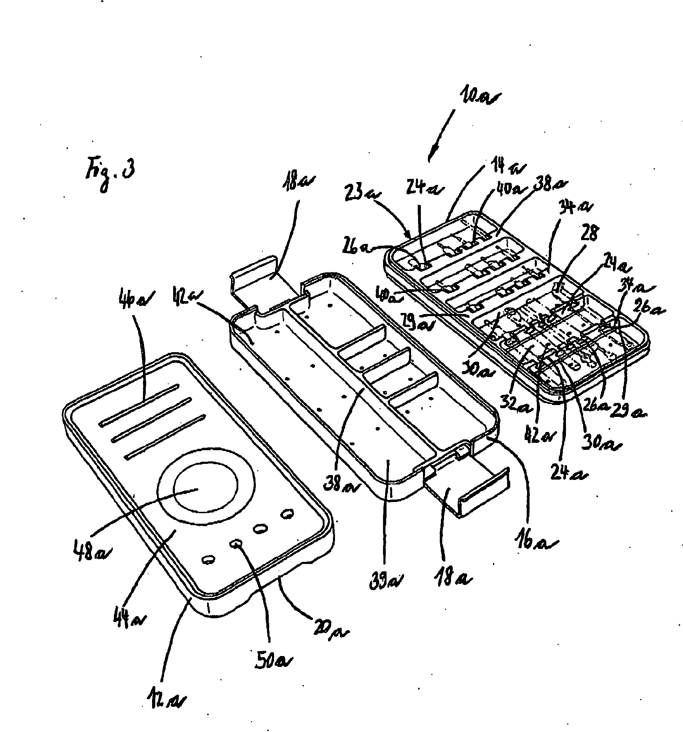

As shown in Figs. 3 and 4 beside the cover 12a also the tray base 32a of the first tray 14a and the tray base 32a of the second tray 16a comprise base perforation to allow even in a closed state of the cassette 10a a sterilization medium to circulate.

The second tray 16a is also provided with inner walls 38a. These inner walls 38a separate five compartments 39a useable for storage of sterilized or already used medical instruments 28.

Furthermore, as shown in Fig. 3 an inner top surface 44a of the cover 12a comprises rips 46a, a bowl-like impression 48a and four blind holes 50a for holding of medical instruments 28 and / or storage of technical elements e.g. during surgery.

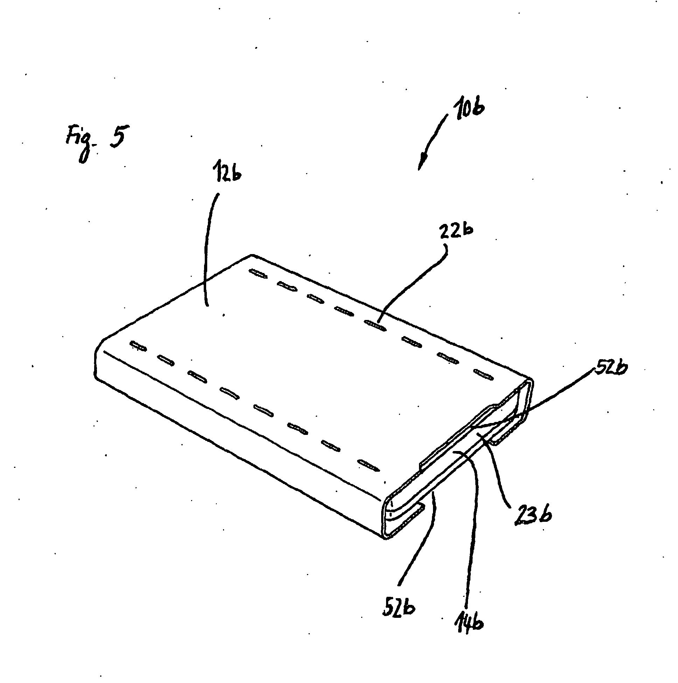

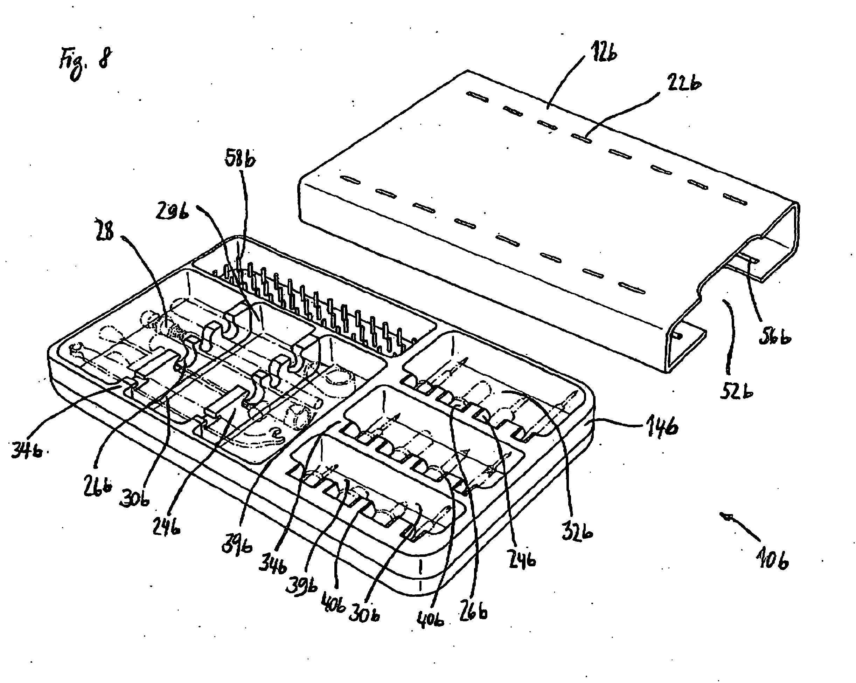

Figures 5 to 8 present a second preferred embodiment of a cassette 10b. The cassette 10b comprises a cover 12b in a sliding-sleeve construction. The cover 12b comprises on its top and bottom side cover perforations 22b which also serve as ventilation holes for a sterilization medium. As shown e.g. in Fig. 8 the cover 12b is provided with indented portions 52b to support opening and closure of the cassette 10b.

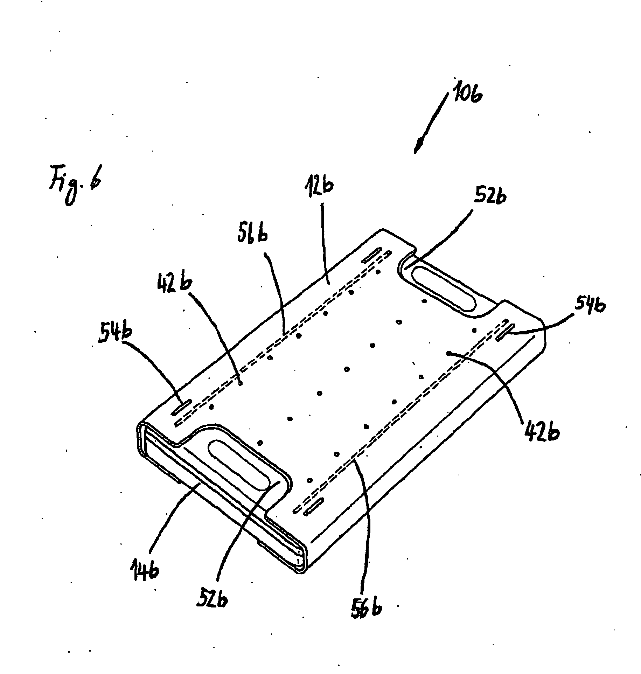

On its bottom side the cover 12b comprises four pedestals 54b at opposing corner regions. As also indicated in Fig. 6 on the inner surface of the bottom side of the cover 12b two rails 56b are formed to support a sliding opening or closure of the cassette 10b.

The tray 14b - housed in the cover 12b of the cassette 10b - comprises similar elements as the first tray 14a of the above described first embodiment of the cassette 10a. Also here comb-like holding means 24b and single-instrument holding means 24b integrally arranged in inner wall cutouts 40b of inner walls 38b are provided. In the tray 14b seven compartments 39B are formed by the comb-like holding means 24b and the inner walls 38b, respectively.

The holding means 24b are of substantially the same construction as the holding means 24a described in connection with the cassette 10a. Also in this embodiment edges and corners of the inner part of the tray 14b and in particular edges and corners between the base socket portion 30b and the tray base 32b are rounded.

In addition, the tray 14b comprises one compartment provided with numerous pins 58b which also serve for storage and holding of medical instruments 28 or technical elements.

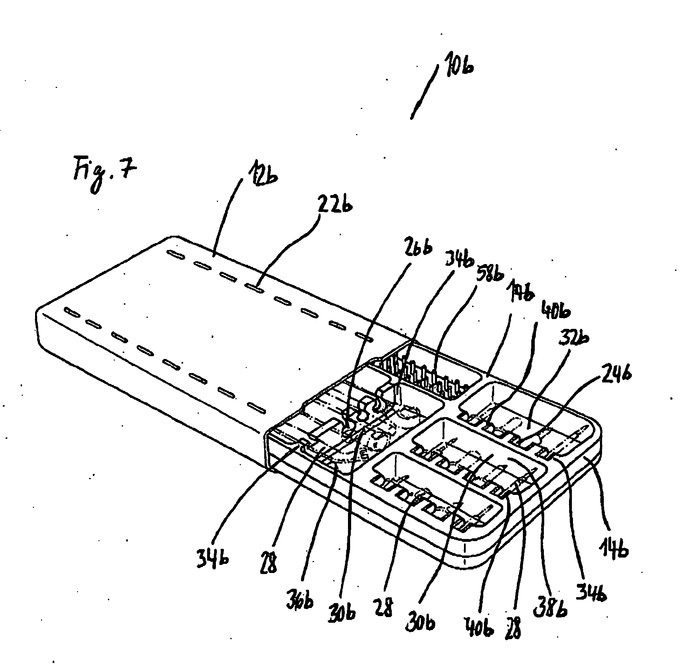

As shown in figures 5 to 8 the cover 12b can close the tray 14b completely (Fig. 5, Fig. 6), partially (Fig. 7) or can be completely separated from the tray 14b (Fig. 8).

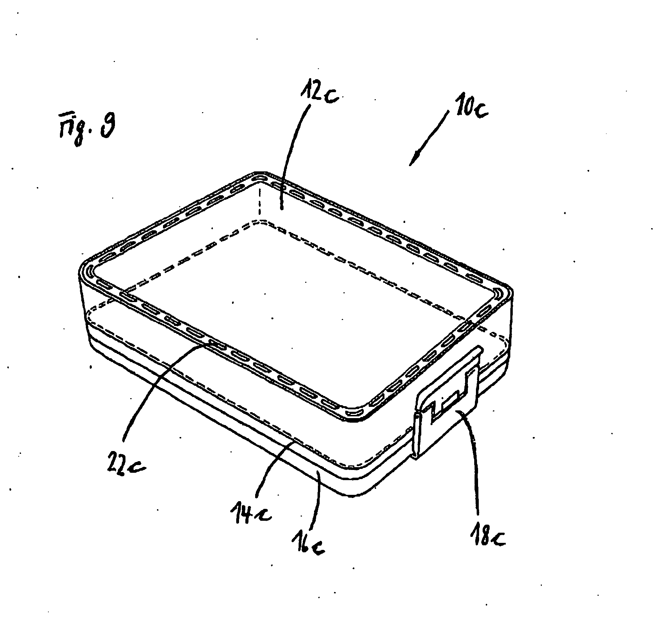

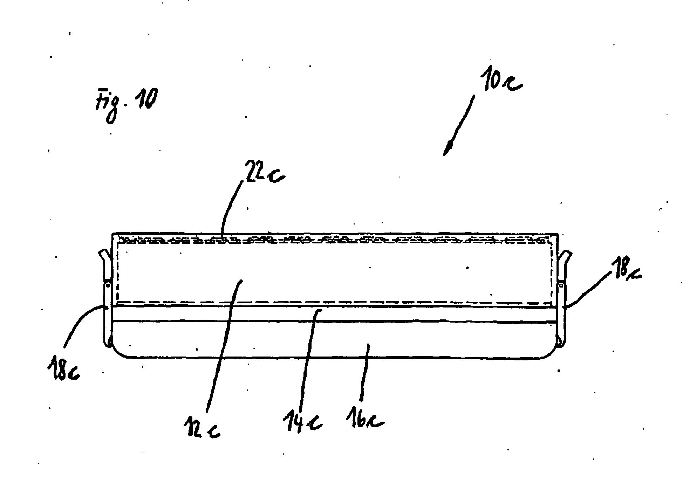

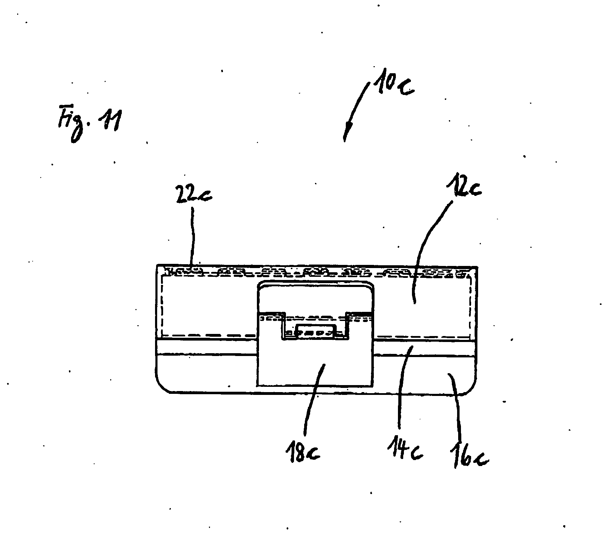

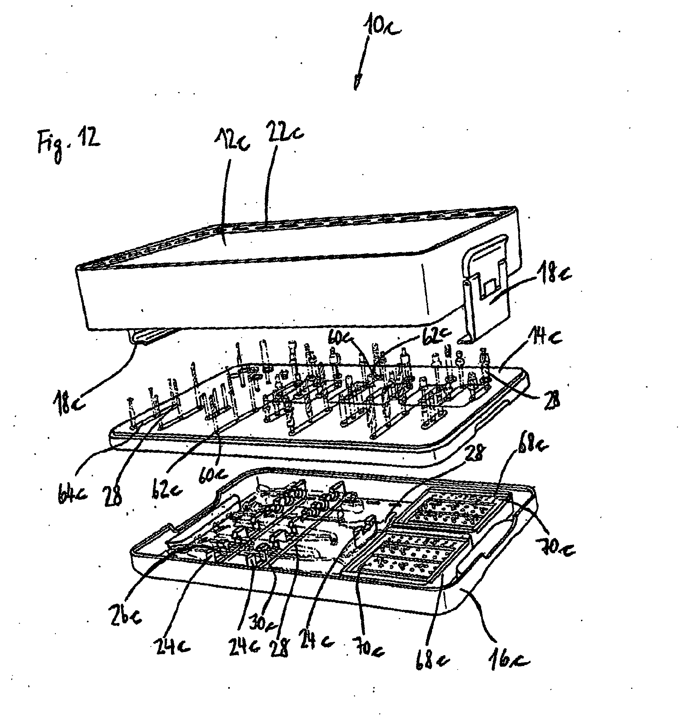

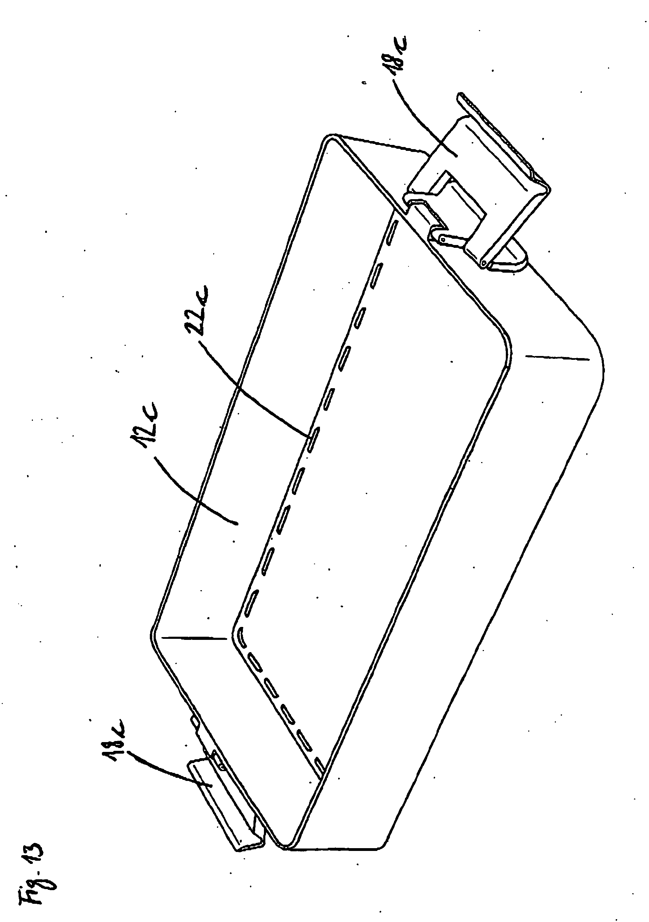

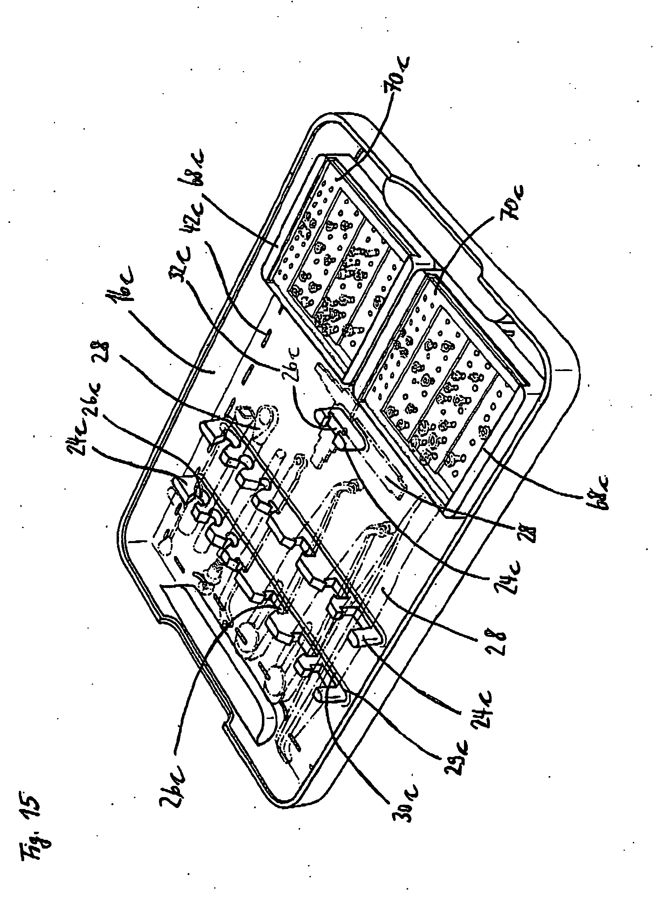

The cassette 10c represents a third preferred embodiment which is shown in figures 9 to 15. Also cassette 10c is of substantially cubical shape with rounded edges. A cover 12c closes a beneath first tray 14c which is arranged above a second tray 16c. By means of clasps 18c the cover 12c is locked to the first tray 14c and the second tray 16c. The clasps 18c engage with impressions 20c of the second tray 16c which are shown for instance in Figs. 12 and 15. Also in this embodiment tilting of the clasps 18c allow an easy closure and opening of the cover 12c from the first and the second tray 14c, 16c. Also in this embodiment the cover 12c can be separated from the below trays 14c, 16c completely.

The cover 12c is provided with a cover perforation 22c which is arranged in an outer circumferential region of the top side of the cover 12c. The cover perforation 22 fulfills the same function as already described in connection with the embodiments of the cassettes 10a and 10b.

As shown in Fig. 12 in an exploded perspective view of the cassette 10c the first tray 14c is formed as a slab and comprises numerous accommodation holes 60c. In these accommodation holes 60c elastomeric grommets 62c are fixedly secured. The grommets 62c are of substantially cylindrical shape and have a central opening in which due to friction-locking shafts of medical instruments 28, in particular drills, are replaceable engaged. To allow sterilization media to get in contact with shafts of the medical instruments 28 a central opening of the grommets 62c can be provided with radially arranged protrusions which surround the central opening. The grommets 62c are preferably arranged within the accommodation holes 60c in the rigid basic structure of the first tray 14c such that a top-side oriented outer end of the grommets 62c is aligned flush with the top surface of the first tray 14c. The grommets 62c are preferably made of a soft resilient plastic material which is proofed to resist sterilization procedures.

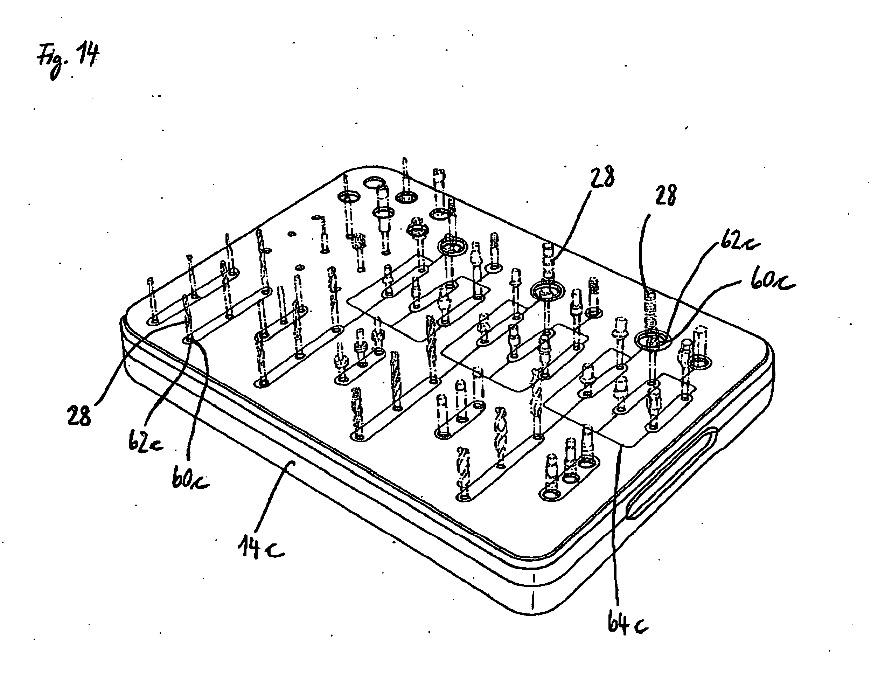

As presented for instance in Fig. 14 the first tray 14c is provided on its top surface with printed information 64c in form of lines, signs, numerals and text which helps to identify the medical instruments 28 and to guide through medical procedures in which these medical instruments 28c are to be used in a certain sequence.

For an easy lift-off of the first tray 14c from the second tray 16c the first and second tray 14c, 16c are provided on their broad sides with matching cutouts to form recessed grips 66c.

In contrast to the first tray 14c the second tray 16c is of box-like design. The second tray 16c comprises two comb-like holding means 24c which are similar to the comb-like holding means 24a and 24b of the first and second embodiment. Also in the third embodiment the rigid basic structure 23c of the second tray 16c forms sockets 29c having base socket portions 30c raised with respect to a tray base 32c. The comb-like holding means 24c are fixed on the top side of said base socket portions 30c. In contrast to the first and the second embodiment the cassette 10c does not comprises lateral socket portions.

The two comb-like holding means 24c of the second tray 16c are also provided with cutouts 26c of substantially rectangular and / or rounded cross-section for medical instruments 28. A further holding means 24c - also fixed on the top side of a base socket portion 30c - is provided with a single cutout 26c for accommodation of only one single medical instrument 28 near the center of the second tray 16c.

Furthermore, the second tray 16c houses two sub-cassettes 68c in which screws, extension pieces and other small technical elements are stored. The sub-cassette 68c can be of same construction as the cassettes 10a, 10b, 10c. In the shown embodiment sub-covers 70c of the sub-cassettes 68c are made of a transparent material and can be provided with printed information on their contents.

Alternatively, the relevant housing space of the second tray 16c can be used for accommodating further retaining means of various type, for instance retaining means apt to hold in place medical instruments for use in guided surgery and similar.

The second tray 16c comprises - similar to the second trays 16a, 16b - based perforations 42c to allow a sterilization medium to flow into the inside of the cassette 10c.

The rigid basic structures 23a, 23b, 23c of the cassettes 10a, 10b, 10c are made of a rigid plastic material of medical grade which can resist standard sterilization procedures. The holding means 24a, 24b, 24c and the grommets 62c are made of a resilient plastic material which allows a plastic deformation for insertion and removal of the medical instruments 28. The holding means 24a, 24b, 24c are fixed to the rigid basic structure 23a, 23b, 23c of the trays 14a, 14b, 16c by molding, welding or gluing in a gap-free manner. Preferably the cassette 10a, 10b, 10c together with the holding means 24a, 24b, 24c and / or grommets 62c are produced in a two-component injection molding method. In addition, all of the above described cassettes 10a, 10b, 10c, in particular the trays 14a, 14b, 14c, 16a, 16b, 16c and the covers 12a, 12b, 12c can be provided with anti-slip means to avoid slipping of these elements if they are placed on slippery surfaces. These anti-slip means are arranged on respective outer surfaces of the elements of the cassettes 10a, 10b, 10c and are comprised of a plastic material, in particular a soft plastic material. The anti-slip means can be formed as complete outer layers or as regions which protrude above the remaining surface of the cassette 10a, 10b, 10c and their elements, respectively.

It should be mentioned that features described in a certain embodiment can also be combined with features discloses in connection with the other embodiments. In addition, shape, size and construction of the holding means 24a, 24b, 24c can be adapted to the respective medical instrument 28 and to specific medical applications.

## Claims

1. A cassette for a medical instrument (28) comprising at least one tray (14a, 14b, 14c, 16c) and a cover (12a, 12b, 12c), the tray (14a, 14b, 14c, 16c) includes a rigid basic structure (23a, 23b, 23c) and fixed thereon a holding means (24a, 24b, 24c) for holding the medical instrument (28), the holding means (24a, 24b, 24c) has a cutout (26a, 26b, 26c) for retaining the medical instrument (28), wherein the holding means (24a, 24b, 24c) is attached to the rigid basic structure (23a, 23b, 23c) of said tray (14a, 14b, 14c, 16c) in a gap-free manner by molding, welding or gluing **characterized in that** said rigid basic structure (23a, 23b, 23c) of said tray (14a, 14b, 16c) forms a socket (29a, 29b, 29c) having a base socket portion (30a, 30b, 30c) raised with respect to a tray base (32a, 32b, 32c) and that said holding means (24a, 24b, 24c) is fixed in the gap-free manner on a top side of said base socket portion (30a, 30b, 30c).

2. The cassette according to claim 1, **characterized in that** edges und corners between the base socket portion (30a, 30b, 30c) and the tray base (32a, 32b, 32c) are rounded.

3. The cassette according to any of claims 1 or 2, **characterized in that** said socket (29a, 29b) of said rigid basic structure (23a, 23b) comprises a lateral socket portion (34a, 34b) protruding from a lateral sidewall (36a, 36b) of the tray (14a, 14b) and that said holding means (24a, 24b) is fixed in the gap-free manner on said lateral socket portion (34a, 34b).

4. The cassette according to any of claims 1 to 3, **characterized in that** the holding means (24a, 24b) is integrated one-sidedly in an inner wall (38a, 38b) of the tray (14a, 14b).

5. The cassette according to any of claims 1 to 4, **characterized in that** a cross-section of the cutout (26a, 26b, 26c) is substantially of a rectangular and / or rounded shape.

6. The cassette according to any of claims 1 to 5, **characterized in that** an elastomeric grommet (62c) is fixed to the rigid basic structure (23c), preferable in a gap-free manner and / or such that an outer end of said grommet (62c) is aligned flush with an inner top surface the tray (14c).

7. The cassette according to any of claims 1 to 6 **characterized in that** the tray (14a, 14b, 14c, 16a, 16c) and / or the cover (12a, 12b, 12c) comprise a perforation (22a, 22b, 22c, 42a, 42b, 42c) to allow circulation of a sterilization medium.

8. The cassette according to any of claims 1 to 7, **characterized in that** the holding means (24a, 24b, 24c) is made of a resilient plastic material which resists sterilization procedures.

9. The cassette according to any of claims 1 to 8, **characterized in that** the tray (16c) houses a sub-cassette (68c) for holding the medical instrument (28) and / or technical elements, where the sub-cassette (68c) is substantially of same construction as the cassette (10a, 10b, 10c).

10. The cassette according to any of claims 1 to 9, **characterized in that** the tray (14c) is provided on its top surface with printed information (64c) in form of lines, signs, numerals and text which helps to identify the medical instruments (28) and to guide through medical procedures in which these medical instruments (28) are to be used in a certain sequence.

11. The cassette according to any of claims 1 to 10, **characterized in that** the cassette (10a, 10b, 10c) and /or the holding means (24a, 24b, 24c) and / or - if provided - grommets (62c) are produced in a two-component injection molding method.

12. The cassette according to any of claims 1 to 11, **characterized in that** the cassette (10a, 10b, 10c), in particular the tray (14a, 14b, 14c, 16a, 16b, 16c) and /or the cover (12a, 12b, 12c), is provided with anti-slip means comprising a plastic material, in particular a soft plastic material.

## Patentansprüche

1. Eine Kassette für ein medizinisches Instrument (28) umfassend mindestens eine Schale (14a, 14b, 14c, 16c) und einen Deckel (12a, 12b, 12c), die Schale (14a, 14b, 14c, 16c) beinhaltet eine starre Grundstruktur (23a, 23b, 23c) und darauf befestigt eine Halteeinrichtung (24a, 24b, 24c) zum Halten des medizinischen Instruments (28), die Halteeinrichtung (24a, 24b, 24c) hat eine Ausnehmung (26a, 26b, 26c) zum Fixieren des medizinischen Instruments (28), wobei die Halteeinrichtung (24a, 24b, 24c) an der starren Grundstruktur (23a, 23b, 23c) der besagten Schale (14a, 14b, 14c, 16c) fugenlos durch Formpressen, Schweissen oder Kleben befestigt ist,
**dadurch gekennzeichnet, dass** die besagte starre Grundstruktur (23a, 23b, 23c) der besagten Schale (14a, 14b, 16c) einen Sockel (29a, 29b, 29c) formt, welche einen bezüglich eines Schalenbodens (32a, 32b, 32c) hervorstehenden Basissockelabschnitt (30a, 30b, 30c) aufweist, und dass besagte Halteeinrichtung (24a, 24b, 24c) fugenlos auf einer Oberseite des besagten Basissockelabschnitts (30a, 30b, 30c) befestigt ist.

2. Die Kassette gemäss Anspruch 1, **dadurch gekennzeichnet, dass** Kanten und Ecken zwischen dem Basissockelabschnitt (30a, 30b, 30c) und dem Schalenboden (32a, 32b, 32c) abgerundet sind.

3. Die Kassette gemäss einem der Ansprüche 1 oder 2, **dadurch gekennzeichnet, dass** besagter Sockel (29a, 29b) der besagten starren Grundstruktur (23a, 23b) einen lateralen Sockelabschnitt (34a, 34b) umfasst, welcher von einer lateralen Seitenwand (36a, 36b) der Schale (14a, 14b) hervorsteht, und dass besagte Halteeinrichtung (24a, 24b) fugenlos auf besagtem lateralen Sockelabschnitt (34a, 34b) befestigt ist.

4. Die Kassette gemäss einem der Ansprüche 1 bis 3,
**dadurch gekennzeichnet, dass** die Halteeinrichtung (24a, 24b) einseitig in eine Innenwand (38a, 38b) der Schale (14a, 14b) integriert ist.

5. Die Kassette gemäss einem der Ansprüche 1 bis 4,
**dadurch gekennzeichnet, dass** ein Querschnitt der Ausnehmung (26a, 26b, 26c) eine im Wesentlichen rechteckige und/oder rundliche Form aufweist.

6. Die Kassette gemäss einem der Ansprüche 1 bis 5,
**dadurch gekennzeichnet, dass** ein elastomerer Dichtring (62c) an der starren Grundstruktur (23c) befestigt ist, vorzugsweise fugenlos und/oder so, dass ein äusseres Ende des besagten Dichtrings (62c) mit einer inneren Oberfläche der Schale (14c) fluchtet.

7. Die Kassette gemäss einem der Ansprüche 1 bis 6,
**dadurch gekennzeichnet, dass** die Schale (14a, 14b, 14c, 16a, 16c) und/oder der Deckel (12a, 12b, 12c) eine Perforation (22a, 22b, 22c, 42a, 42b, 42c) aufweist, um eine Zirkulation eines Sterilisationsmediums zu erlauben.

8. Die Kassette gemäss einem der Ansprüche 1 bis 7,
**dadurch gekennzeichnet, dass** die Halteeinrichtung (24a, 24b, 24c) aus einem elastischen Kunststoffmaterial besteht, das Sterilisationsprozessen widersteht.

9. Die Kassette gemäss einem der Ansprüche 1 bis 8,
**dadurch gekennzeichnet, dass** die Schale (16c) eine Unterkassette (68c) beherbergt zum Halten des medizinischen Instruments (28) und/oder von technischen Elementen, wobei die Unterkassette (68c) im Wesentlichen von derselben Konstruktion ist wie die Kassette (10a, 10b, 10c).

10. Die Kassette gemäss einem der Ansprüche 1 bis 9,
**dadurch gekennzeichnet, dass** die Schale (14c) auf ihrer Oberfläche mit aufgedruckten Informationen (64c) in Form von Linien, Zeichen, Nummern und Text versehen ist, welche helfen, die medizinischen Instrumente (28) zu identifizieren und durch medizinische Arbeitsabläufe zu führen, in welchen diese medizinischen Instrumente (28) in einer bestimmten Abfolge anzuwenden sind.

11. Die Kassette gemäss einem der Ansprüche 1 bis 10,
**dadurch gekennzeichnet, dass** die Kassette (10a, 10b, 10c) und/oder die Halteeinrichtung (24a, 24b, 24c) und/oder - wenn vorhanden - Dichtringe (62c) in einem Zweikomponenten-Spritzverfahren hergestellt sind.

12. Die Kassette gemäss einem der Ansprüche 1 bis 11,
**dadurch gekennzeichnet, dass** die Kassette (10a, 10b, 10c), insbesondere die Schale (14a, 14b, 14c, 16a, 16b, 16c) und/oder der Deckel (12a, 12b, 12c) mit einem Gleitschutzmittel, umfassend ein Kunststoffmaterial, insbesondere ein Weichkunststoffmaterial, ausgestattet sind.

## Revendications

1. Cassette pour un instrument médical (28), comprenant au moins un plateau (14a, 14b, 14c, 16c) et un couvercle (12a, 12b, 12c), le plateau (14a, 14b, 14c, 16c) comportant une structure élémentaire rigide (23a, 23b, 23c) et, fixé sur celle-ci, un moyen de maintien (24a, 24b, 24c) destiné à maintenir l'instrument médical (28), le moyen de maintien (24a, 24b, 24c) étant pourvu d'une découpe (26a, 26b, 26c) destinée à retenir l'instrument médical (28), le moyen de maintien (24a, 24b, 24c) étant attaché à la structure élémentaire rigide (23a, 23b, 23c) dudit plateau (14a, 14b, 14c, 16c) sans laisser d'interstices par moulage, soudage ou collage,
**caractérisée en ce que** ladite structure élémentaire rigide (23a, 23b, 23c) dudit plateau (14a, 14b, 16c) forme une alvéole (29a, 29b, 29c) pourvue d'une partie d'alvéole formant socle (30a, 30b, 30c) surélevée par rapport à un socle de plateau (32a, 32b, 32c) et **en ce que** ledit moyen de maintien (24a, 24b, 24c) est fixé sans laisser d'interstices sur un côté de dessus de ladite partie d'alvéole formant socle (30a, 30b, 30c).

2. Cassette selon la revendication 1, **caractérisée en ce que** des bords et des coins entre la partie d'alvéole formant socle (30a, 30b, 30c) et le socle de plateau (32a, 32b, 32c) sont arrondis.

3. Cassette selon l'une quelconque des revendications 1 ou 2, **caractérisée en ce que** ladite alvéole (29a, 29b) de ladite structure élémentaire rigide (23a, 23b) comprend une partie d'alvéole latérale (34a, 34b) faisant saillie d'une paroi latérale (36a, 36b) du plateau (14a, 14b) et **en ce que** ledit moyen de maintien (24a, 24b) est fixé sans laisser d'interstices sur ladite partie d'alvéole latérale (34a, 34b).

4. Cassette selon l'une quelconque des revendications 1 à 3, **caractérisée en ce que** le moyen de maintien (24a, 24b) est intégré d'un seul côté dans une paroi intérieure (38a, 38b) du plateau (14a, 14b).

5. Cassette selon l'une quelconque des revendications 1 à 4, **caractérisée en ce qu'**une section transversale de la découpe (26a, 26b, 26c) est de forme sensiblement rectangulaire et/ou arrondie.

6. Cassette selon l'une quelconque des revendications 1 à 5, **caractérisée en ce qu'**un oeillet élastomère (62c) est fixé à la structure élémentaire rigide (23c), de préférence sans laisser d'interstices et/ou de telle sorte qu'une extrémité extérieure dudit oeillet (62c) soit alignée de façon affleurante avec une surface intérieure de dessus du plateau (14c).

7. Cassette selon l'une quelconque des revendications 1 à 6, **caractérisée en ce que** le plateau (14a, 14b, 14c, 16a, 16c) et/ou le couvercle (12a, 12b, 12c) comprend/comprennent une perforation (22a, 22b, 22c, 42a, 42b, 42c) pour permettre la circulation d'un milieu de stérilisation.

8. Cassette selon l'une quelconque des revendications 1 à 7, **caractérisée en ce que** le moyen de maintien (24a, 24b, 24c) est constitué d'une matière plastique élastique qui résiste à des procédures de stérilisation.

9. Cassette selon l'une quelconque des revendications 1 à 8, **caractérisée en ce que** le plateau (16c) abrite une sous-cassette (68c) destinée à maintenir l'instrument médical (28) et/ou des éléments techniques, la sous-cassette (68c) possédant sensiblement la même structure que la cassette (10a, 10b, 10c).

10. Cassette selon l'une quelconque des revendications 1 à 9, **caractérisée en ce que** la surface de dessus du plateau (14c) est dotée d'informations imprimées (64c) sous forme de lignes, de signes, de chiffres et de texte qui facilitent l'identification des instruments médicaux (28) et l'accompagnement dans des procédures médicales faisant appel à ces instruments médicaux (28) suivant une certaine séquence.

11. Cassette selon l'une quelconque des revendications 1 à 10, **caractérisée en ce que** la cassette (10a, 10b, 10c) et/ou le moyen de maintien (24a, 24b, 24c) et/ou - le cas échéant - les oeillets (62c) sont fabriqués par un procédé de moulage par injection sandwich.

12. Cassette selon l'une quelconque des revendications 1 à 11, **caractérisée en ce que** la cassette (10a, 10b, 10c), et plus particulièrement le plateau (14a, 14b, 14c, 16a, 16b, 16c) et/ou le couvercle (12a, 12b, 12c), sont dotés de moyens antidérapants comprenant un matériau plastique, plus particulièrement un matériau plastique souple.
